# EUROPEAN PATENT APPLICATION

(11) **EP 4 588 480 A1**
(43) Date of publication of application: **23.07.2025**
(21) Application number: 23865249.9
(22) Date of filing: 28.08.2023
(51) Int. Cl.: A61K 31/685, A61K 39/39, A61K 45/00, A61P 35/00, A61P 37/04, A61P 43/00, C12N 5/0784

(54) **ANTI-TUMOR IMMUNE RESPONSE ENHANCER**

(30) Priority: 12.09.2022 JP 2022144313
(71) Applicant: National Center for Child Health and Development, Tokyo 157-8535 (JP); Hasumi, Kenichiro, Tokyo 167-0051 (JP)
(72) Inventor: RII, Ko, Tokyo 157-8535 (JP); HASUMI, Kenichiro, Tokyo 167-0051 (JP)
(74) Representative: Script Intellectual Property LLP
(86) International application number: PCT/JP2023/030929
(87) International publication number: WO 2024/057892

(57) **Abstract**

The present invention aims to provide means to maintain and enhance the anti-tumor activity of TADC in a tumor microenvironment. The present inventors found that when a composition comprising three kinds of phospholipids "cPLs adjuvant" is administered, the degree of growth of tumors implanted to mice was notably suppressed, and confirmed that immature dendritic cells, when cultured *in vitro* in the presence of the cPLs adjuvant, become mature bone marrow-derived dendritic cells and induce cytokine production.

## Description

### Technical Field

The present invention relates to an anti-tumor immune response enhancer, and more specifically relates to an anti-tumor immune response enhancer comprising phosphatidylcholine, phosphatidylethanolamine, and phosphatidylserine.

### Background Art

Dendritic cells (DC) are known as most powerful antigen presenting cells capable of priming both naive T cells and memory T cells and inducing antigen-specific anti-tumor immune (see for example, Non-patent Document 1). However, DC in a tumor microenvironment, that is, tumor-associated dendritic cells (hereinafter, also referred to as TADC), are known likely to cause immunosuppression or immune tolerance such as low expression of costimulatory molecules, representing an immature phenotype (see for example, Non-patent Document 2), representing a suppressive dysfunctional phenotype, and likely contributing to the escape of cancer cells from the immune surveillance network of a host (see for example, Non-patent Document 3). Further, TADC has been reported to suppress the activation of anti-tumor type T cells and secreting various types of cytokines that accelerate the growth of tumor cells (see for example, Non-patent Document 4). Thus, in a tumor microenvironment, DC is known to be potentially a double-edged sword that acts positively and negatively on the anti-tumor response (see for example, Non-patent Document 5), and considered likely to reduce an anti-tumor activity in a conventional cancer immunotherapy.

On the other hand, a method for activating TIL is proposed (see for example, Patent Document 1), which comprises administering a fermentation composition generated via fermentation in a culture medium of symbiotic microflora to a subject in need of activating tumor infiltrating lymphocytes (TIL). Also proposed is a use of a composition for dendritic cell activation *ex vivo,* comprising one or more lipids having at least one cationic lipid and at least one antigen (see for example, Patent Document 2), and a use of all-trans retinoic acid injection (see for example, Patent Document 3), that enables activity reduction of abnormal bone marrow-derived immunosuppressive cells in a tumor patient, differentiation induction of bone marrow-derived immunosuppressive cells, and suppression of tumor growth and recurrence.

### Prior Art Documents

### Patent Documents

Patent Document 1: Japanese unexamined Patent Application Publication No. 2021-517587
Patent Document 2: Japanese unexamined Patent Application Publication No. 2022-36961
Patent Document 3: Japanese unexamined Patent Application Publication (Translation of PCT Application) No. 2019-528315

### Non-patent Documents

Non-patent Document 1: Steinman RM, Banchereau J. Taking dendritic cells into medicine. Nature. 2007;449(7161):419-26
Non-patent Document 2: Oncotarget. 2016;7(39):63204-14
Non-patent Document 3: J Cancer. 2013;4(1):36-44
Non-patent Document 4: J Leukoc Biol. 2017;102(2):317-324
Non-patent Document 5: Frontiers in Oncology 2013 Volume 3 Article 90:1-12

### Summary of the Invention

### Object to be Solved by the Invention

The present invention aims to provide means to maintain and enhance the anti-tumor activity of TADC in a tumor microenvironment.

### Means to Solve the Object

The present inventors have been studying on various components, with focusing on a tumor microenvironment of mice, to maintain and enhance the anti-tumor activity of TADC, which is considered immature DC, and found that when a composition comprising three kinds of phospholipids, which are phosphatidylcholine (PC), phosphatidylethanolamine (PE), and phosphatidylserine (PS), (Combined PhosphoLipids adjuvant: cPLs adjuvant) (hereinafter, referred also to as "cPLs adjuvant") is administered after tumors implanted to mice have grown to a predetermined size, the degree of growth of tumors implanted to mice was notably suppressed. Additionally, the present inventors confirmed that when immature DC are cultured *in vitro* in the presence of the cPLs adjuvant, such cultured DC becomes mature bone marrow-derived dendritic cells (BMDCs) and induce inflammatory cytokine production. Further, the present inventors removed the tumor tissues from the cancer cell-implanted mice to which the cPLs adjuvant was administered and analyzed for the character of tumor filtrating leukocytes in the tumor tissues, and confirmed that the markers expressed by the mature DC were expressed, and inflammatory cytokine production was accelerated with the acceleration or T cell production, whereby the present invention has come to accomplish.

### Effect of the Invention

Administration of the anti-tumor immune response enhancer of the present invention can suppress the degree of growth of tumors, also induce immature bone marrow-derived dendritic cells and TADC to mature, and induce immature dendritic cells to mature *in vitro* by culturing in the presence of the anti-tumor immune response enhancer.

That is, the present invention is as follows.
[1] An anti-tumor immune response enhancer comprising phosphatidylcholine, phosphatidylethanolamine, and phosphatidylserine.
[2] The anti-tumor immune response enhancer according to the above [1], used for growth suppression of a tumor in a subject.
[3] The anti-tumor immune response enhancer according to the above [1], used for inducing an immature dendritic cell to be a mature dendritic cell.
[4] The anti-tumor immune response enhancer according to the above [3], used for inducing an immature dendritic cell to be CD11b⁺ CD11c⁺ viable cells.
[5] The anti-tumor immune response enhancer according to the above [4], wherein the immature dendritic cell is an immature conventional dendritic cell or a tumor-associated dendritic cell.
[6] The anti-tumor immune response enhancer according to the above [1] or [2], containing 50 to 90% phosphatidylcholine, 5 to 25% phosphatidylethanolamine, and 5 to 25% phosphatidylserine.
[7] The anti-tumor immune response enhancer according to the above [1] or [2], further comprising one or more anticancer agents.
[8] A method for preparing a mature dendritic cell, comprising culturing an immature dendritic cell *in vitro* in the presence of the anti-tumor immune response enhancer according to the above [1] or [2].

Further, the present invention also comprises: an agent comprising phosphatidylcholine, phosphatidylethanolamine, and phosphatidylserine for a used in an anti-tumor immune response enhancement therapy; a cytokine production accelerator comprising phosphatidylcholine, phosphatidylethanolamine, and phosphatidylserine; a combination of phosphatidylcholine, phosphatidylethanolamine, and phosphatidylserine used to enhance an anti-tumor immune response; a method for enhancing an anti-tumor immune response in a subject, comprising administering an anti-tumor immune response enhancer comprising phosphatidylcholine, phosphatidylethanolamine, and phosphatidylserine to a subject; a method for enhancing an anti-tumor immune response comprising a step of administering phosphatidylcholine, phosphatidylethanolamine, and phosphatidylserine to a subject in need of enhancing an anti-tumor immune response; a method for activating tumor-associated dendritic cells in a subject, comprising a step of administering an anti-tumor immune response enhancer comprising phosphatidylcholine, phosphatidylethanolamine, and phosphatidylserine to a subject in need of enhancing an anti-tumor immune response in a tumor microenvironment, a method for administering an agent for reducing immunosuppressive property of a tumor microenvironment in a subject and enhancing clinical utility, a cancer immunotherapy using activated mature dendritic cells by an anti-tumor immune response enhancer, activated tumor-associated dendritic cells by an anti-tumor immune response enhancer, or activated mature dendritic cells by an anti-tumor immune response enhancer, and a cancer immunotherapy using activated tumor-associated dendritic cells by an anti-tumor immune response enhancer.

### Brief Description of Drawings

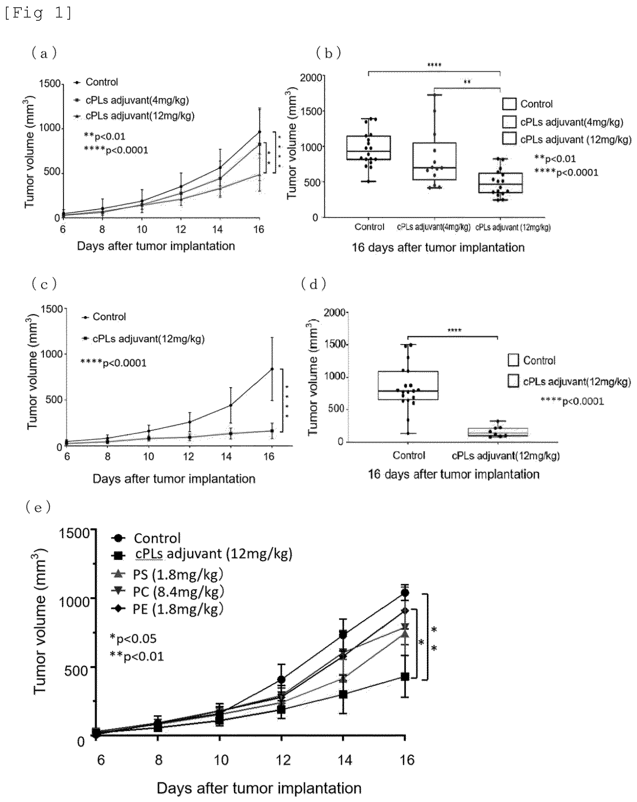

[Figure 1] (a) is a graph showing changes in the tumor volume up to 16 days after implantation in the mice to which the cPLs adjuvant was subcutaneously injected into the flank in a dose of 4 mg/kg of mouse body weight or 12 mg/kg of mouse body weight after MO4-Luc cell implantation, and control mice to which the cPLs adjuvant was not administered, respectively. The horizontal axis shows the number of days elapsed after implantation in the mice, and the vertical axis shows the tumor volume of the MO4-Luc cell-implanted mice. (b) is a tumor volume histogram on day 16 after implantation in the mice to which the cPLs adjuvant was subcutaneously injected in a dose of 4 mg/kg of mouse body weight or 12 mg/kg of mouse body weight, and control mice to which the cPLs adjuvant was not administered, respectively. (c) is a graph showing changes in the tumor volume up to 16 days after implantation in the mice to which the cPLs adjuvant was subcutaneously injected into the flank in a dose of 12 mg/kg of mouse body weight after C26 cell implantation, and control mice to which the cPLs adjuvant was not administered, respectively. (d) is a tumor volume histogram on day 16 after implantation in the mice to which the cPLs adjuvant was administered in a dose of 12 mg/kg of mouse body weight, and control mice to which the cPLs adjuvant was not administered, respectively. (e) is a graph showing changes in the tumor volume up to 16 days after implantation when the cPLs adjuvant, PC, PE, and PS were respectively administered to the MO4-Luc cell-implanted mice.
[Figure 2] Figure 2 shows the flow cytometric analysis after mouse conventional immature bone marrow-derived dendritic cells were cultured in the presence of the cPLs adjuvant.
[Figure 3] Figure 3 is graphs showing expressions of cell surface markers (a) IA/IE, (b) CD80, and (c) CD40 respectively in the delta (Δ) value of mean fluorescence intensity (MFI) after mouse conventional immature bone marrow-derived dendritic cells were cultured in the presence of the cPLs adjuvant.
[Figure 4] Figure 4 is graphs showing expressions of (a) IL-1β, (b) IL-12, and (c) IL-6 respectively in the delta value of MFI on imDC cultured for 2 days in the presence of the cPLs adjuvant.
[Figure 5] Both (a) and (b) show the flow cytometric analyses of TIL of cPLs adjuvant-treated groups.
[Figure 6] Figure 6 is graphs showing expressions of (a) CD86 and (b) IA/IE respectively in the delta value of MFI in TILs of cPLs adjuvant-treated groups.
[Figure 7] Figure 7 is graphs showing expressions of (a) IL-1β, (b) IL-12, and (c) IFN-γ in the delta value of MFI in TILs of cPLs adjuvant-treated groups.
[Figure 8] Figure 8 is graphs showing productions of (a) IFN-γ, (c) TNF-α, and (e) IL-2 in CD4⁺ T cells in TILs, and productions of (b) IFN-γ, (d) TNF-α, and (f) IL-2 in CD8⁺ T cells in the delta value of MFI.

### Mode of Carrying Out the Invention

The anti-tumor immune response enhancer of the present invention is not particularly limited as long as it is an agent comprising phosphatidylcholine (PC), phosphatidylethanolamine (PE), and phosphatidylserine (PS) and capable of enhancing an anti-tumor immune response, and examples include an agent free of phospholipids other than PC, PE, and PS, and an agent free of phospholipids other than PC, PE, and PS as an essential component. Examples of the administration subject for the above anti-tumor immune response enhancer include mammals such as mouse, rat, sheep, pig, cow, cat, dog, monkey, and human.

Examples of the method for producing the anti-tumor immune response enhancer of the present invention include a method for producing the anti-tumor immune response enhancer by dissolving and mixing PC, PE, and PS sequentially, or as a mixture, in a solvent, and examples of the above solvent include any solvent capable of providing the effects of the present invention and non-invasive to a subject, such as preferably ethanol. Examples of the concentration of solution include 0.5 to 2 mg/mL, and preferably 0.8 to 1.5 mg/mL, of PC, PE, and PS, and examples of the ratio of PC, PE, and PS contained in such a solution include 50 to 90% PC, 5 to 25% PE, and 5 to 25% PS, preferably 60 to 80% PC, 10 to 20% PE, and 10 to 20% PS, and more preferably 65 to 75% PC, 12.5 to 17.5% PE, and 12.5 to 17.5% PS.

Examples of the above PC include naturally derived PC such as soybean-derived PC, and egg yolk-derived PC, and synthetic PC comprising saturated or unsaturated carboxylic acids having 7 to 22 carbon atoms. Examples of the synthetic PC specifically include dilauroyl PC, dimyristoyl PC, dioleoyl PC, dipalmitoyl PC, palmitoleoyl PC, and distearoyl PC. Additionally, the fatty acid residue part bound to the 1st and 2nd positions of glycerin is the same or different.

Examples of the above PE include naturally derived PE such as soybean-derived PE, and soybean-derived hydrogenated PE, and synthetic PE such as PE comprising saturated or unsaturated carboxylic acids having 7 to 22 carbon atoms. Examples specifically include dilauroyl PE, dimyristoyl PE, dipalmitoyl PE, dioleoyl PE, palmitoleoyl PE, and distearoyl PE. Additionally, the fatty acid residue part bound to the 1st and 2nd positions of glycerin is the same or different.

Examples of the above PS include naturally derived PS such as soybean-derived PS, and soybean-derived hydrogenated PS, and synthetic PS such as PS comprising saturated or unsaturated carboxylic acids having 7 to 22 carbon atoms. Examples specifically include dilauroyl PS, dimyristoyl PS, dipalmitoyl PS, dioleoyl PS, palmitoleoyl PS, and distearoyl PS. Additionally, the fatty acid residue part bound to the 1st and 2nd positions of glycerin is the same or different.

The tumors in the present invention are not particularly limited as long as tumors whose growth is suppressed when the anti-tumor immune response enhancer of the present invention is administered, with malignant tumors being preferable, and examples of the malignant tumor include hematopoietic cell malignant tumor, head and neck cancer, brain tumor, breast cancer, uterine cancer, cervical cancer, ovarian cancer, esophageal cancer, stomach cancer, appendix cancer, colorectal cancer, liver cancer, gallbladder cancer, bile duct cancer, pancreatic cancer, kidney cancer, adrenal cancer, gastrointestinal stromal tumor, mesothelioma, thyroid cancer, lung cancer, osteosarcoma, bone cancer, prostate cancer, testicular tumor, bladder cancer, skin cancer, and anal cancer.

The anti-tumor immune response enhancer of the present invention can be used for growth suppression of a tumor in a subject, and examples of the growth suppression of a tumor include, when administering the anti-tumor immune response enhancer of the present invention, the reduction in the degree of a tumor volume increase, the reduction of a tumor volume, and tumor disappearance, in comparison with the case where the anti-tumor immune response enhancer of the present invention is not administered. The case of reducing the degree of a tumor volume increase is not particularly limited, and examples include, when calculated as tumor volume (mm³) = 1/2(tumor length × (tumor width)²), a case where a tumor volume reduces after a predetermined time elapsed from administration of the anti-tumor immune response enhancer of the present invention, in comparison with a tumor volume with no such administration, and examples specifically include a case where a tumor volume with such administration is 4/5 or less, preferably 3/5 or less, and more preferably 1/2 or less, of a tumor volume with no such administration.

The anti-tumor immune response enhancer of the present invention can be used for inducing immature dendritic cells to be mature dendritic cells, and preferably used for inducing immature dendritic cells to be CD11b⁺CD11c⁺ viable cells. Examples of the immature dendritic cell include immature conventional bone marrow-derived dendritic cell, and TADC present in the body, but these cells can also be prepared *in vitro.*

The method for preparing the above immature conventional dendritic cells *in vitro* is not particularly limited as long as it is a known method, and examples include a method for preparing bone marrow-derived immature conventional dendritic cells by collecting bone marrow cells of the thigh bone and/or shin bone of a mammal to be an administration subject such as mouse, rat, sheep, pig, cow, cat, dog, monkey, or human, and culturing the collected bone marrow cells in RPMI-1640 culture medium comprising FBS, Penicillin-Streptomycin, sodium pyruvate, and MEM non-essential amino acids solution, GlutaMAX-I, 2-mercaptoethanol, IL-4, and GM-CSF, and in the case of mouse cells, examples preferably include a method for preparing bone marrow-derived immature conventional dendritic cells by culturing in RPMI-1640 culture medium comprising 10% FBS, 1% Penicillin-Streptomycin, 1% sodium pyruvate, and a 1% MEM non-essential amino acids solution, 1% GlutaMAX-I, 50 µM of 0.4% 2-mercaptoethanol, 10 ng/mL of mouse IL-4, and 10 ng/mL of GM-CSF.

Examples of the method for preparing mature dendritic cells *in vitro* by culturing immature dendritic cells include a method for culturing *in vitro* immature dendritic cells collected from a subject in the presence of the anti-tumor immune response enhancer of the present invention, and examples include a method for culturing in a culture medium to which 0.1 to 10 mg/mL, preferably 0.5 to 5 mg/mL, more preferably 0.8 to 1.6 mg/mL, and further preferably 1.0 to **1.4** mg/mL of the anti-tumor immune response enhancer is added for 12 hours to 72 hours, preferably 24 hours to 72 hours, and more preferably 36 hours to 60 hours.

The above anti-tumor immune response enhancer can be used for preparing mature dendritic cells by culturing *in vitro* immature dendritic cells collected from a subject, and the *in vitro* prepared mature dendritic cells can also be administered into the body of the subject.

Examples of the method for preparing *in vitro* the above TADC is not particularly limited as long as it is a known method for isolating TADC present in a tumor microenvironment, and examples include a method in which a tumor removed from the body of a mammal is washed with PBS and finely cut using a scalpel, the cut tumor tissues are further dispersed to prepare a single-cell suspension, and the tumor in the form of a single-cell suspension is filtered, washed with PBS, and centrifuged by Percoll gradient to isolate mononuclear cells. The thus prepared TADC can be evaluated *in vitro* on the degree of growth suppression of a tumor.

The above tumor microenvironment is an *in vivo* environment in which cells other than malignant tumor (cancer) cells are included in a solid tumor or coexist around a solid tumor, and examples include an environment having characteristics of accelerating the growth of tumor cells, accelerating tumor metastasis, and/or avoiding host immunity, and where various cells such as dendritic cells are present, and the immune against tumors are suppressed.

Examples of the method for confirming that the anti-tumor immune response enhancer of the present invention has induced immature dendritic cells such as immature conventional dendritic cells and TADC to have been mature dendritic cells include a method for confirming that test cells are determined to have been mature dendritic cells when detecting markers expressed by mature dendritic cells or activated cytokines by a means capable of carrying out several parametric analyses on physical and chemical properties of individual cell such as flow cytometry.

Examples of the method for confirming that immature conventional dendritic cells have matured by the anti-tumor immune response enhancer of the present invention include confirming that the expressions of cell surface markers such as CD (Cluster of Differentiation) 11b, CD11c, IA/IE, CD80, and CD40 by the above parametric analyses are determined to have been more significantly increased than a case where the anti-tumor immune response enhancer of the present invention is not administered (added), and/or that production levels of cytokines such as interleukin (IL)-1β, IL-12, and IL-6 are determined to have been more significantly increased by matured and activated conventional dendritic cells than a case where the anti-tumor immune response enhancer of the present invention is not administered (added).

Example of the method for confirming that TADC has matured by the anti-tumor immune response enhancer of the present invention include that expression levels of cell surface markers such as CD11b, CD11c, CD86, and IA/IE in CD45 viable cells are determined to have been more significantly increased by the above parametric analyses than a case where the anti-tumor immune response enhancer of the present invention is not administered (added). Additionally, examples include confirming that production levels of cytokines such as IL-1β, IL-12, and IFN-γ are determined to have been more significantly increased by matured and activated conventional dendritic cells than a case where the anti-tumor immune response enhancer of the present invention is not administered (added).

Examples of the method for confirming that TADC has matured by the anti-tumor immune response enhancer of the present invention further include confirming that production levels of IFN-γ and IL-2 in CD4-positive TADC have been more significantly increased than a case where the anti-tumor immune response enhancer of the present invention is not administered (added), and a production level of TNF-α has been more significantly reduced than a case where the anti-tumor immune response enhancer of the present invention is not administered.

Additionally, examples of the method for confirming that TADC has matured by the anti-tumor immune response enhancer of the present invention further include confirming that production levels of IFN-γ, IL-2, and TNF-α in CD8-positive TADC have been more significantly increased than a case where the anti-tumor immune response enhancer of the present invention is not administered (added).

The administration subject for the anti-tumor immune response enhancer of the present invention is preferably a tumor-carrying subject, but the enhancer can also be administered even to a non-tumor-carrying subject as a preventive measure for preventing a (malignant) tumor from developing.

The administration mode of the anti-tumor immune response enhancer of the present invention is not particularly limited as long as it provides the effects of the present invention, while parenteral administration that administers by subcutaneous injection, intramuscular injection, or intravenous injection, etc. are preferable, and in the case where a location of a solid tumor can be identified, the administration to the solid tumor, and the administration around the solid tumor and near the solid tumor are preferable.

The dose of the anti-tumor immune response enhancer of the present invention is not particularly limited as long as it provides the effects of the present invention and does not cause severe side effects and, for example, for deciding a dose of the anti-tumor immune response enhancer of the present invention for human, a dose for human can be decided by, as usually practiced in the art, the HED conversion method of extrapolating a dosage at which the equivalent action is expressed in human from a body surface area of a test animal such as mouse, or accumulating experiment data by a known method such as examining data of maximum blood concentration (Cmax) and area under the concentration-time curve (AUC).

The anti-tumor immune response enhancer of the present invention, when used in combination with other anticancer agents, can enhance anticancer effects of the anticancer agents. Examples of the other anticancer agents include alkylating agents such as cyclophosphamide, bendamustine, ifosfamide, and dacarbazine; antimetabolites such as pentostatin, fludarabine, cladribine, methotrexate, 6-mercaptopurine, and enocitabine; molecular targeted agents such as rituximab, cetuximab, and trastuzumab; kinase inhibitors such as imatinib, gefitinib, erlotinib, afatinib, dasatinib, sunitinib, and trametinib; calcineurin inhibitors such as cyclosporine and tacrolimus; anticancer antibiotics such as idarubicin, doxorubicin, and mitomycin C; plant alkaloids such as irinotecan and etoposide; platinum-based agents such as cisplatin, oxaliplatin, and carboplatin; and immunoregulatory agents such as interferon, nivolumab, and pembrolizumab.

In the present description, the terms such as "increased", "increase", "enhance", or "activate" are all used herein to mean an increase in statistically significant level. In some embodiments, the terms "increased", "increase", "enhance", or "activate (activated action due to an increase)" may mean, in comparison with a level of the control, an increase of at least 10%, for example, including an increase of at least about 20%, or at least about 30%, or at least about 40%, or at least about 50%, or at least about 60%, or at least about 70%, or at least about 80%, or at least about 90%, or more, including an increase of 100%, or, in comparison with a control level, an any increase between 10 to 100%, or an increase of at least about 2 times, or at least about 3 times, or at least about 4 times, or at least about 5 times, or at least about 10 times, or an any increase between 2 times and 10 times or more.

### (Statistical analysis method)

In the present description, the term "statistically significant" or "significantly" refers to statistical significance, and the statistical significance can be set at p < 0.05.

Hereinafter, the present invention will be more specifically described in reference to examples, but the technical scope of the present invention is not limited to these examples.

### Examples

### [Example 1]

### (Preparation of lipid composition)

A solution was prepared by dissolving three kinds of phospholipids, phosphatidylcholine (PC) (Soy PC (95%), manufactured by Avanti Polar Lipids, Inc), phosphatidylethanolamine (PE) (Lipoid S PE, (soybean-derived purified phosphatidylethanolamine, 98% or more), manufactured by Lipoid GmbH), and soybean-derived phosphatidylserine (PS) (Lipamine (R) PS 90 PN, manufactured by Nagase ChemteX Corporation), in EtOH in a mass ratio of PC:PE:PS = 14:3:3. Specifically, 0.84 mg of PC, 0.18 mg of PE, and 0.18 mg of PS were dissolved in 1 mL of ethanol to prepare 1.2 mg/mL of lipid composition cPLs adjuvant.

### (Mouse)

Eight-week-old female C57BL/6NCrSlc mice (hereinafter referred to as "B6 mouse") and 8-week-old female Balb/c mice were purchased from Japan SLC, Inc. Each mouse was kept with filtered water and feed starting from 1 week before the experiment. The mice were kept in conformity with the National Center for Child Health and Development (NCCHD) guidelines on laboratory animal welfare.

### (Preparation of cancer cell line)

OVA-expressing mouse melanoma (hereinafter referred to as "MO4-Luc cell") that expresses firefly luciferase extended by Dr. Jun Fang, Faculty of Pharmaceutical Sciences, Sojo University and mouse colon cancer cell line, C26 cell, were cultured respectively at 37°C under 5% CO₂ concentration in RPMI1640 culture medium (manufactured by Wako Pure Chemical Industries, Ltd.) to which 10% fetal bovine serum and 1% Penicillin-Streptomycin (manufactured by Thermo Fisher Scientific Inc.).

### (Statistical analysis method)

Each of the results shown in the examples was analyzed as mean ± standard deviation (SD). Data was analyzed using GraphPad Prism software (version 7.0, manufactured by GraphPad Software, Inc.). For the comparison between 2 groups (normal distribution), one-sided non-paired (independent sample), or paired (in 3 independent experiments) student's t-test was used. For the comparison between 2 groups, one-sided Wilcoxon matched-pairs signed-rank test was used (abnormal distribution). For the comparison among several groups, One Way Analysis of Variance (One-way ANOVA) using Tukey's multiple comparison test was used. The wording "significantly" refers to statistical significance. The wording "notably" means extremely big significant difference. The statistical significance was set at p < 0.05.

### (Implantation of MO4-Luc cells to mouse)

The above cultured MO4-Luc cells were washed twice with PBS and resuspended in PBS so as to be a concentration of 3 × 10⁶ cells/mL. The resuspended MO4-Luc cells were subcutaneously injected into the shaved right flank of the above B6 mice (3 × 10⁵/100 µL s.c.) to prepare MO4-Luc cell-implanted mice (day 0 after implantation).

For the above MO4-Luc cell-implanted mice, a tumor length and width were measured using a Vernier scale every 2 days starting from day 6 after MO4-Luc cell implantation thereby to calculate a tumor volume. The calculation formula for the tumor volume was tumor volume (mm³) = 1/2 (tumor length × (tumor width)²). On day 8 after MO4-Luc cell implantation, MO4-Luc cell-implanted mice having 100 mm³ > tumor volume > 10 mm³ were selected, to which the cPLs adjuvant was administered. That is, on day 8, day 11, and day 14 after MO4-Luc cell implantation, the cPLs adjuvant in a dose of 4 mg/kg of mouse body weight or 12 mg/kg of mouse body weight was intraperitoneally injected. Mice to which the cPLs adjuvant was not administered were defined as control mice, and tumor volumes were measured in the same manner. A graph showing changes in the tumor volume up to 16 days after implantation (**p < 0.01, ****p < 0.0001) is shown in Figure 1(a), and a tumor volume histogram on day 16 after MO4-Luc melanoma implantation as mean ± SD by One Way Analysis of Variance (One-way ANOVA) using Tukey's multiple comparison test (**p < 0.01, ****p < 0.0001) is shown in Figure 1(b).

### (Results)

As is obvious from Figure 1(a), in the MO4-Luc cell-implanted mice, the degrees of tumor volume increase in the mice to which the cPLs adjuvant was administered in a dose of 12 mg/kg of mouse body weight and mice to which the cPLs adjuvant was administered in a dose of 4 mg/kg of mouse body weight were evidently smaller than the degrees of tumor volume increases in the non-treated control mice. In Figure 1(a), the tumor volumes of mice to which the cPLs adjuvant was administered in a dose of 12 mg/kg of mouse body weight on day 16 after MO4-Luc cell implantation were about 1/2 the tumor volumes of the non-treated control mice which were notably small. The tumor volumes of mice to which the cPLs adjuvant was administered in a dose of 12 mg/kg of mouse body weight were about 3/5 the tumor volumes of the mice to which the cPLs adjuvant was administered in a dose of 4 mg/kg of mouse body weight which were significantly small.

As is obvious from Figure 1(b), on day 16 after MO4-Luc cell implantation, notably significant differences were shown in the tumor volumes of the mice to which the cPLs adjuvant was administered in a dose of 12 mg/kg of mouse body weight and the non-treated control mice. Additionally, significant differences were also found in the tumor volumes of the mice to which the cPLs adjuvant was administered in a dose of 12 mg/kg of mouse body weight and the mice to which the cPLs adjuvant was administered in a dose of 4 mg/kg of mouse body weight.

From the above results, it was confirmed that, in the MO4-Luc cell-implanted mice, the cPLs adjuvant administration provides excellent growth suppression action on a tumor.

### [Example 2]

### (Implantation of C26 cells to mouse)

The above cultured C26 cells were washed twice with PBS and resuspended in PBS so as to be a concentration of 3 × 10⁶ cells/mL. The resuspended C26 cells were subcutaneously injected into the shaved right flank of the above Balb/c mice (3 × 10⁵/100 µL s.c.) to prepare C26 cell-implanted mice (day 0 after implantation).

For the above C26 cell-implanted mice, a tumor length and width were measured using a Vernier scale every 2 days starting from day 6 after C26 cell implantation thereby to calculate a tumor volume as in the above MO4-Luc cell-implanted mice. On day 8 after cell implantation, C26 cell-implanted mice having 100 mm³ > tumor volume > 10 mm³ were selected, to which, on day 8, day 11, and day 14 after implantation, the cPLs adjuvant in a dose of 4 mg/kg of mouse body weight or 12 mg/kg of mouse body weight was intraperitoneally injected. The mice to which the cPLs adjuvant was not administered were defined as control mice, and tumor volumes were similarly measured. As in Example 1, a graph showing changes in the tumor volume (****p < 0.0001) is shown in Figure 1(c), and a histogram (****p < 0.0001) is shown in Figure 1(d).

### (Results)

As is obvious from Figure 1(c), in the C26 cell-implanted mice, the degrees of tumor volume increases in the mice to which the cPLs adjuvant was administered in a dose of 12 mg/kg were evidently smaller than the degrees of tumor volume increases in the non-treated control mice. In Figure 1(c), the tumor volumes of mice to which the cPLs adjuvant was administered in a dose of 12 mg/kg of mouse body weight on day 16 after C26 cell implantation were about 1/4 the tumor volumes of the non-treated control mice which were notably small. Additionally, as is obvious from Figure 1(d), when the tumor volumes were compared between the mice to which the cPLs adjuvant was administered in a dose of 12 mg/kg of mouse body weight and the non-treated control mice on day 16 after C26 cell implantation, it was shown that the size of tumors in the mice to which the cPLs adjuvant was administered was notably smaller.

From the above results, it was confirmed that, in the C26 cell-implanted mice, the cPLs adjuvant administration provides excellent growth suppression action on a tumor.

### [Example 3]

### (Synergistic effect of cPLs adjuvant)

A PC solution produced by dissolving 0.84 mg of PC in 1 mL of ethanol, a PE solution produced by dissolving 0.18 mg of PE in 1 mL of ethanol, and a PS solution produced by dissolving 0.18 mg of PS in 1 mL of ethanol were respectively produced, 8.4 mg/kg of mouse body weight of PC, 1.8 mg/kg of mouse body weight of PE, or 1.8 mg/kg of mouse body weight of PS was administered to the MO4-Luc cell-implanted mice, respectively, in place of the cPLs adjuvant in the dose of 12 mg/kg of mouse body weight in accordance with the above procedure of Example 1. Figure 1(e) is a graph (*p < 0.05, **p < 0.01) showing changes in the tumor volume up to 16 days after implantation in each mouse.

As is obvious from Figure 1(e) showing changes in the tumor volume in the MO4-Luc implanted mice, the degree of tumor volume increases in the mice to which the cPLs adjuvant was administered in a dose of 12 mg/kg of mouse body weight in the MO4-Luc cell-implanted mice was smaller than the degree of tumor volume increases in the non-treated control mice and in the mice to which each of PC, PE, or PS was individually administered. Additionally, in Figure 1(e), the size of tumors on day 16 after implantation in the MO4-Luc mice to which the cPLs adjuvant was administered in a dose of 12 mg/kg of mouse body weight was less than 1/2 the tumor volume of the non-treated control mice which was notably small, and significantly smaller than the tumor volume of the PE-administered mice, whereby the excellent anti-tumor effect of the cPLs adjuvant was shown.

### [Example 4]

### [Character analysis of conventional dendritic cell]

For more detail investigation of the cPLs adjuvant effect on DC, first the cPLs adjuvant effect on bone marrow-derived immature conventional dendritic cells (DC) was investigated.

### (Preparation of immature mouse bone marrow-derived dendritic cell)

Bone marrow cells of the thigh bone and shin bone were collected from 8- to 12-week-old B6 mice, and immature conventional mouse bone marrow dendritic cells (hereinafter also referred to as "imDC") were prepared from the collected bone marrow cells. That is, B6 mouse-derived bone marrow cells were cultured for 5 days in RPMI-1640 culture medium comprising 10%FBS, 1% Penicillin-Streptomycin (manufactured by Gibco Thermo Fisher Scientific Inc.), 1% sodium pyruvate (manufactured by Gibco Thermo Fisher Scientific Inc.), and 1%MEM non-essential amino acids solution (manufactured by Gibco Thermo Fisher Scientific Inc.), 1% GlutaMAX-I (manufactured by Gibco Thermo Fisher Scientific Inc.), 50 µM of 0.4% 2-mercaptoethanol (manufactured by Wako Pure Chemical Industries, Ltd.), 10 ng/mL of mouse IL-4 (manufactured by PeproTech), and 10 ng/mL of GM-CSF (manufactured by BioLegend Inc.), thereby to prepare imDC.

### (Addition of cPLs adjuvant to mouse bone marrow-derived immature dendritic cell)

The above imDC was further cultured for 2 more days in a 24-well plate to which 1.2 mg/mL of the cPLs adjuvant was further added, the cells were collected, and the number of cells was counted. The cell viability was 90% or more.

### (Flow cytometric analysis on dendritic cells after cPLs adjuvant addition)

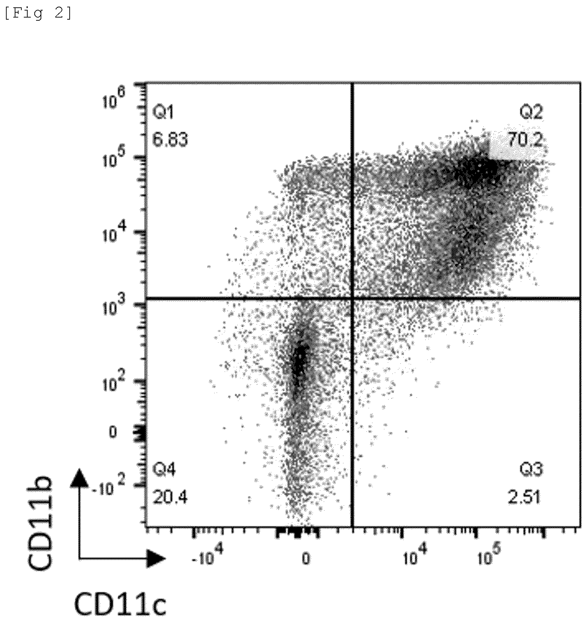

The cells cultured for 2 days in the presence of the cPLs adjuvant were analyzed by flow cytometry. The cells cultured for 2 days in the presence of the cPLs adjuvant were suspended, after washed, in PBS at an optimal concentration. For cytokine staining in the cell, the cells were stimulated in 50 ng/mL of PMA (Phorbor 12-myristate 13-acetate) and 500 ng/mL of ionomycin (manufactured by Sigma-Aldrich Co. LLC) in the presence of a Brefeldin A solution (manufactured by Thermo Fisher Scientific Inc.) at 37°C for 4 hours. Subsequently, for easier intracellular staining, permeabilization was achieved using intracellular fixation/permeabilization buffer (cat#00-5523-00, manufactured by Thermo Fisher Scientific Inc.). The flow cytometric analysis results are shown in Figure 2.

In the flow cytometric analysis hereafter, the analysis was carried out using a cell analyzer LE-SP6800 (manufactured by Sony Corporation) or BD FACSymphony (TM) (manufactured by BD Biosciences), and FlowJo software (version 10.5.0; manufactured by BD Biosciences).

### (Staining reagent)

Hereafter, the following reagents were used for the intracellular staining.
For staining, LIVE/DEAD(TM) Fixable Aqua Dead Cell Stain Kit, for 405 nm excitation (L34957, manufactured by Thermo Fisher Scientific Inc.), mouse Fc Blocking Reagent (130-092-575, manufactured by Miltenyi Biotec), and anti-mouse monoclonal antibodies (all are products of BioLegend Inc.) shown below which bind to any of Brilliant Violet605, Alexa Fluor 700, APC/Cyanine7, PE/Cyanine7, PE/Dazzle594, PerCP/Cy5.5, APC, PE, and FITC were used.
CD11b (M1/70),
CD11c (N418),
CD40 (3/23),
CD80 (16-10A1),
CD86 (GL-1),
IA/IE (M5/114.15.2),
CD8 (53-6.7),
CD4 (RM4-5),
CD45 (30-F11),
IL-10 (JES5-16E3),
IL-12 (C15.6),
IL-6 (MP5-20F3),
IL-1β (NJTEN3),
TNF-α (MP6-XT22),
IFN-γ (XMG1.2).

### (Results)

As is obvious from Figure 2, it was confirmed that CD11b⁺ and CD11c⁺, which are presented in the conventional dendritic cells (cDC), were expressed after the above imDC was cultured for 2 days in the presence of the cPLs adjuvant. Thus, it was confirmed that the mouse immature DC, when cultured in the presence of the cPLs adjuvant, is induced to be mature mouse bone marrow dendritic cells (mature BMDC), in which CD11b⁺ and CD11c⁺ are expressed.

### (Expression of markers)

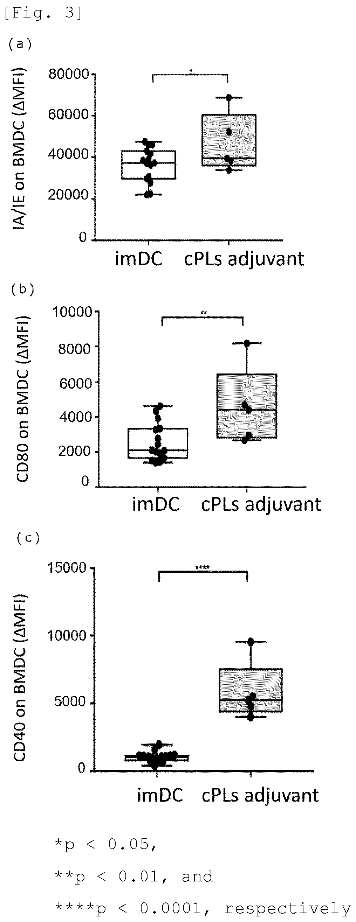

Figure 3 shows graphs showing expressions of (a) IA/IE, (b) CD80, and (c) CD40, the surface markers of the above mature BMDC, respectively in the delta value of MFI.

### (Results)

It was confirmed that mature BMDC (horizontal axis: cPLs adjuvant) cultured for 2 days in the presence of the cPLs adjuvant had more significantly increased expressions of IA/IE, CD80, and CD40 than the cPLs adjuvant non-treated imDC (horizontal axis: imDC). Additionally, it was also confirmed that CD86, when culturing imDC for 2 days in the presence of the cPLs adjuvant, has a tendency to increase the expression.

The above results showed that imDC sufficiently matures and becomes mature BMDC in the presence of the cPLs adjuvant, and the expressions of IA/IE antigen and CD40 increase, whereby the anti-tumor immune enhancement effect can be provided.

### (Cytokine production)

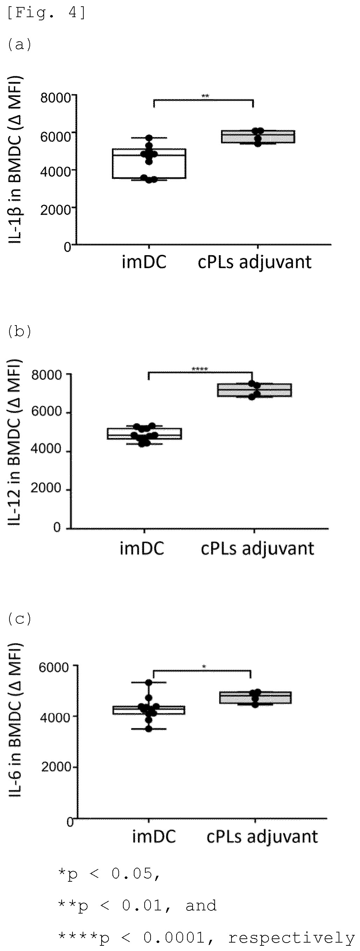

Figure 4 is graphs respectively showing productions of (a) IL-1β, (b) IL-12, and (c) IL-6 in the delta value of MFI on DC, which was cultured for 2 days in the presence of the above cPLs adjuvant and became mature BMDC.

### (Results)

It was confirmed that imDC cultured for 2 days in the presence of the cPLs adjuvant (horizontal axis: cPLs adjuvant) had more significantly increased productions of IL-1β, IL-12, and IL-6 than the cPLs adjuvant non-treated imDC (horizontal axis: imDC).

The above results showed that the secretion of IL-1β, which is a cytokine that activates T cells and accelerates antigen recognition, increased, and the secretion of IL-12, which is a cytokine that induces Th1 cells and controls an inflammatory response, increased. Additionally, the secretion of IL-6, which is an inflammatory cytokine, also increased. These results showed that imDC sufficiently matures and becomes mature BMDC in the presence of the cPLs adjuvant, and the expressions of IL-1β, IL-12, and IL-6 increase, whereby the anti-tumor immune enhancement effect can be provided.

### [Example 5]

### [Character analysis of tumor-associated dendritic cell (TADC)]

For evaluating the effect of the cPLs adjuvant on TADC normally present in a tumor of TILs, TILs were isolated from tumor tissues to analyze markers and the like presented on TADC.

### (Preparation of tumor filtrating leukocyte)

Tumor filtrating leukocytes were respectively prepared from tumors removed 16 days lapsed from implantation of the MO4-Luc cell-implanted mice to which the cPLs adjuvant (12 mg/kg of mouse body weight) was administered or non-treated such mice to which the adjuvant was not administered in Example 1. The removed tumors were washed with PBS, finely cut using a scalpel, and the cut mouse tumor tissues were further dispersed using a Miltenyi Tumor Dissociation Kit (Cat No.130-096-730, manufactured by Miltenyi Biotec) and GentleMACS Octo dissociator (Cat No.130-093-235, manufactured by Miltenyi Biotec) to prepare a single-cell suspension. The tumor in the form of a single-cell suspension was filtered using a 70 µM pre-separation filter (manufactured by Thermo Fisher Scientific Inc.), washed with PBS, and centrifuged (600 × g) by Percoll gradients (55% and 70%, GE Healthcare) at 20°C for 20 minutes to isolate mononuclear cells, and each TILs was prepared.

### (Analysis on surface markers of TADC in TILs)

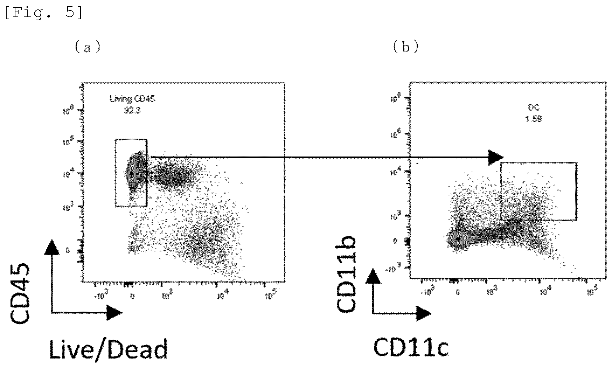

The markers expressed on TILs of the MO4-Luc cell-implanted mice to which the cPLs adjuvant (12 mg/kg) was administered were analyzed by the flow cytometry by the procedure described in the above Example 4. The results are shown in Figure 5.

### (Results)

As is obvious from the flow cytometric analysis of Figure 5, in TILs prepared from the tumors removed from the mice to which the cPLs adjuvant was administered, TADC were shown as CD45⁺ CD11b⁺ CD11c⁺ viable cells. Thus, it is confirmed that the markers of mature dendritic cells were expressed as in the markers of the mature conventional dendritic cells confirmed in the above Example 4.

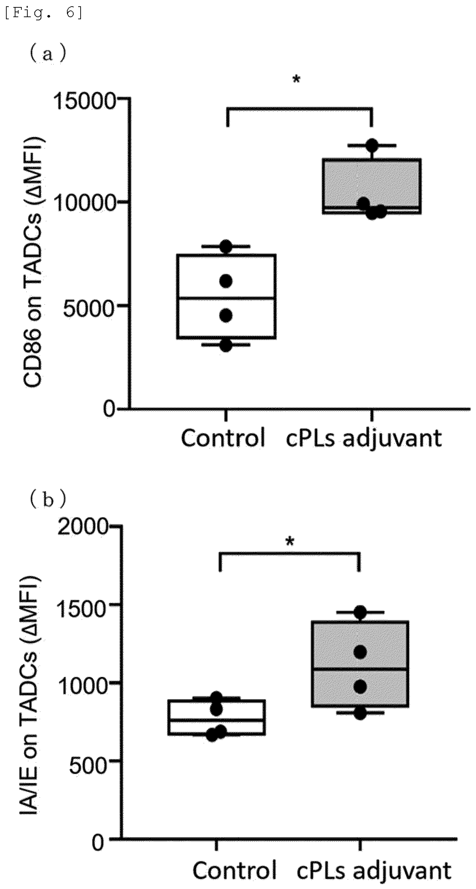

Figure 6 is graphs showing expressions of (a) CD86 and (b) IA/IE respectively in the delta value of MFI on TADC in the above TILs.

It was confirmed that the expressions of CD86 and IA/IE, which are mature markers of dendritic cells, were significantly increased in TILs prepared from the tumors removed from the mice to which the above cPLs adjuvant was administered. Thus, it is shown that the cPLs adjuvant has become capable of accelerating maturity of TADC in TILs and providing the anti-tumor immune enhancement effect, thereby suggesting that dendritic cells sufficiently mature even in a tumor microenvironment by the cPLs adjuvant and became capable of providing the anti-tumor immune enhancement effect.

### (Cytokine production)

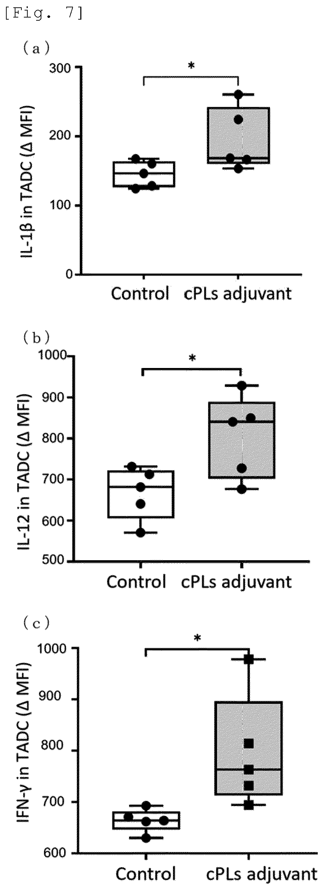

The functionality of T cell in TILs was evaluated from the viewpoint of cytokine production. Figure 7 is graphs respectively showing expressions of the cytokines (a) IL-1β, (b) IL-12, and (c) IFN-γ in the delta value of MFI.

### (Results)

In TILs (horizontal axis: cPLs adjuvant) of the above cPLs adjuvant-treated groups, it was confirmed that the productions of IL-1β, IL-12, and IFN-γ was more significantly increased in TIL prepared from the tumors removed from the mice to which the cPLs adjuvant was administered than TILs (horizontal axis: control) of the cPLs adjuvant-non-treated control groups.

In TADC prepared from the tumors removed from the mice to which the above cPLs adjuvant was administered, it was confirmed that the secretions of IL-1β, IL-12, and IFN-γ, which are cytokines that accelerate antigen recognition and T cell activation, increased, and the TADC maturity in TILs is accelerated by the cPLs adjuvant. Thus, it was suggested that dendritic cells sufficiently mature even in a tumor microenvironment by the cPLs adjuvant and became capable of providing the anti-tumor immune enhancement effect.

The productions of (a) IFN-γ, (c) TNF-α, and (e) IL-2 in CD4⁺ T cells in TILs, and the productions of (b) IFN-γ, (d) TNF-α, and (f) IL-2 in CD8⁺ T cells were calculated in the delta value of MFI. The results are shown in Figure 9, respectively.

### (Results)

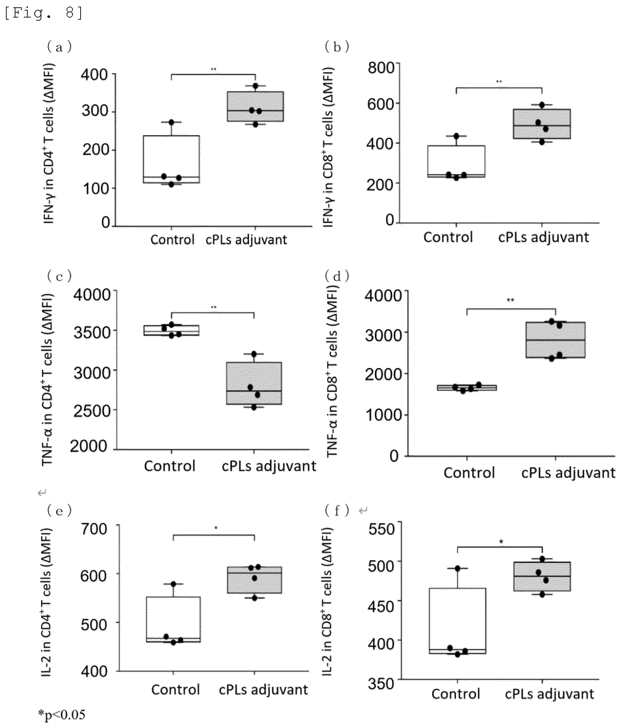

As is obvious from Figure 8, in CD4⁺ T cells (horizontal axis: cPLs adjuvant) of the cPLs adjuvant-treated groups, the production levels of IFN-γ and IL-2 were significantly higher than the cPLs adjuvant non-treated CD4⁺ T cells (horizontal axis: control). On the other hand, the production level of TNF-α in CD4⁺ T cells of the cPLs adjuvant-treated groups was significantly lower than in CD4⁺ T cells of the cPLs adjuvant-non-treated control groups.

The above results showed that the activated CD4⁺ helper T cell (Th) increases the cytokine productions of IL-2, IFN-γ and the like, and CD4⁺ T cell has been activated in a tumor microenvironment by the administration of cPLs adjuvant.

In CD8⁺ T cells of the cPLs adjuvant-treated groups, the production levels of IFN-γ, IL-2, and TNF-α were significantly higher than the cPLs adjuvant-non-treated CD8⁺ T cells.

It was confirmed that the cPLs adjuvant enhances the expressions of IFN-γ, IL-2, and TNF-αin CD8⁺ T cells. It was suggested that the anti-tumor immune effect was enhanced by the administration of cPLs adjuvant in a tumor microenvironment.

### (Conclusion)

It was suggested that one of the mechanisms for releasing the immune tolerance in a tumor microenvironment is the activation of T cells caused by the cPLs adjuvant administration.

## Claims

1. An anti-tumor immune response enhancer comprising phosphatidylcholine, phosphatidylethanolamine, and phosphatidylserine.

2. The anti-tumor immune response enhancer according to claim 1, used for growth suppression of a tumor in a subject.

3. The anti-tumor immune response enhancer according to claim 1, used for inducing an immature dendritic cell to be a mature dendritic cell.

4. The anti-tumor immune response enhancer according to claim 3, used for inducing an immature dendritic cell to be CD11b⁺ CD11c⁺ viable cells.

5. The anti-tumor immune response enhancer according to claim 4, wherein the immature dendritic cell is an immature conventional dendritic cell or a tumor-associated dendritic cell.

6. The anti-tumor immune response enhancer according to claim 1 or 2, containing 50 to 90% phosphatidylcholine, 5 to 25% phosphatidylethanolamine, and 5 to 25% phosphatidylserine.

7. The anti-tumor immune response enhancer according to claim 1 or 2, further comprising one or more anticancer agents.

8. A method for preparing a mature dendritic cell, comprising culturing an immature dendritic cell *in vitro* in the presence of the anti-tumor immune response enhancer according to claim 1 or 2.
